# EUROPEAN PATENT SPECIFICATION

(11) **EP 2 007 766 B1**
(45) Date of publication and mention of the grant of the patent: **09.03.2011**
(21) Application number: 07754689.3
(22) Date of filing: 28.03.2007
(51) Int. Cl.: C07D 473/04

(54) **NARCOTINE PURIFICATION PROCESS**
NARKOTINREINIGUNGSVERFAHREN
PROCÉDÉ DE PURIFICATION DE LA NARCOTINE

(30) Priority: 11.04.2006 US 791012 P
(43) Date of publication of application: 31.12.2008
(73) Proprietor: MALLINCKRODT, INC., Hazelwood, MO 63042 (US)
(72) Inventor: TOMAZI, Keith, G., Florissant, Missouri 63031 (US); LOVE JR., Leroy, St. Louis, Missouri 63034 (US); HAAR JR., Joseph P., Edwardsville, Illinois 62025 (US)
(74) Representative: Adam, Holger
(86) International application number: PCT/US2007/008205
(87) International publication number: WO 2007/120538

(56) References cited:
- P. CHANDRA: "Recovery, Separation and Purification of Narcotine and Papaverine from Indian Opium" BULLETIN ON NARCOTICS, vol. 33, no. 1, 1981, pages 55-64, XP009089931 cited in the application

## Description

### FIELD OF THE INVENTION

The present invention relates to a process for purifying Narcotine and, more particularly, to a process for the removal of color and impurities in Narcotine to form Noscapine.

### BACKGROUND OF THE INVENTION

"Narcotine" is a name by which an impure form of "Noscapine" is known. Noscapine is indentified by CAS Registry No. 128-62-1 and Noscapine Hydrochloride is identified by CAS Registry No. 912-60-7. Worldwide demand for Noscapine has increased over the last few years. However, due to color and impurities only a fraction of produced Narcotine can be purified sufficiently to form Noscapine. In addition, there is an impurity known as a "Noscapine Analog," CAS Registry No. 220875-41-2, thought to originate in the Opium starting material, which may be present in Narcotine up to 1.5%. To meet the guidelines set forth by the International Conference on Harmonisation of Technical Requirements for Registration of Pharmaceuticals for Human Use (ICH) the amount of the Noscapine Analog must be reduced below 0.15%.

U.S. Patent Application No. 10/648,781 (Pub. No. 2005/0049278) relates to methods of making and using Noscapine derivatives. The application discloses that separation and/or purification of the derivatives involves adding water or an aqueous acid solution, such as hydrochloric acid, acetic acid, citric acid or sulfuric acid. If an acid is used, the aqueous layer is preferably rendered alkaline (i.e., a pH of at least 8) by adding an effective amount of a suitable base, such as sodium carbonate. The resulting mixture may then be extracted with a suitable organic solvent, such as diethyl ether, dichloromethane, and/or ethyl acetate.

Ramanathan, V.S. et al., Bulletin on Narcotics 33(1), 55-64 (1981) discloses several processes for purifying narcotine, including the separation of narcotine from a narcotine-papaverine mixture, derived from opium, using an aqueous sodium hydroxide solution. In one process, the narcotine-papaverine mixture is placed in sodium hydroxide and heated to 80-85°C, cooled and filtered to remove the insoluble papaverine. The clear filtrate is acidified to pH 3 with 40% sulphuric acid and heated to 90°C and allowed to cool. A sodium hydroxide solution is then added to the solution to raise the pH to 10 resulting in a precipitate of white crystalline narcotine.

Sim, S. K. "Medicinal Plant Alkaloids," 2nd Ed. Toronto Press 1970 discloses a step-wise process for the separation of the six major alkaloids of opium (i.e., morphine, codeine, thebaine, papaverine, narceine and noscapine). Each of the alkaloids is removed sequentially from a starting solution made from opium powder. Noscapine is the last alkaloid isolated from the solution using ammonia to make the solution alkaline, dissolving the resulting precipitate in boiling alcohol and then crystallization.

U.S. Patent No. 1,947,330 relates to a process for producing compositions of alkaloids of opium by extracting morphine and narcotine. According to the disclosed process, 100 parts of raw opium powder are macerated for 12 hours with 500 parts cold water. The solution is heated to 65°C for 1/2 hour and contacted with ice. The residue is then macerated with 7 parts of dilute phosphoric acid and 250 parts water for 6 hours. After several iterations, the extract is dissolved in a mixture of 10 grams alcohol, 10 grams water and 5 grams ammonia. The solution is left to stand for 48 hours and filtered with 40% alcohol and washed with water to provide a residue that contains morphine and narcotine.

However, there is a need in the art to a more effective process for purifying Narcotine to form Noscapine. In particular, there is a need in the art to provide a process for the removal of color and impurities, including the removal of a Noscapine Analog, to form Noscapine.

### SUMMARY OF THE INVENTION

The present invention relates to a process for purifying Narcotine and, more particularly, to a process for the removal of color and impurities in Narcotine to form Noscapine.

According to the present invention, a process for the removal of impurities from a Narcotine product includes adding an aqueous isopropanol solution to the Narcotine product wherein the isopropanol concentration of the solution is about 20% to about 70% by volume, preferably about 30% to about 60%, most preferably about 30% to about 50%; and adjusting the pH of the solution with a strong base to a pH of about 10 to about 14, wherein impurities are removed from the Narcotine product.

### BRIEF DESCRIPTION OF THE DRAWINGS

Figure 1 shows the steps of an exemplary purification process;

Figure 2 shows an interaction plot comparing isopropanol concentration and pH of exemplary solutions that may be used in the process of the present invention; and

Figure 3 shows an interaction plot comparing isopropanol concentration and pH of exemplary solutions that may be used in the process of the present invention.

### DETAILED DESCRIPTION

The present invention relates to a process for purifying Narcotine and, more particularly, to a process for the removal of color and impurities in Narcotine to form Noscapine.

Narcotine products that may be purified using the process of the present invention may be extracted from an Opium starting material. One skilled in the art will appreciate that a number of methods may be used to extract the Narcotine product from Opium. For example, the Narcotine product may be extracted from Opium, and separated from Morphine and Codeine, by a multistage extraction.

According to the present invention, a process for the removal of impurities from a Narcotine product comprises: forming an aqueous isopropanol solution with the Narcotine product in suspension wherein the isopropanol concentration of the solution is about 20% to about 70% by volume, preferably about 30% to about 60%, most preferably about 30% to about 50%; and adjusting the pH of the solution with a strong base to a pH of about 10 to about 14, preferably about 12 to about 13, wherein impurities are removed from the Narcotine product.

In some embodiments, the isopropanol concentration of the solution is about 35% to about 55% by volume. In other embodiments, the isopropanol concentration of the solution is about 40% to about 50% by volume.

One skilled in the art will appreciate that any strong base known in the art may be used to adjust the pH of the solution. Sodium hydroxide or its solutions, potassium hydroxide or its solutions, calcium hydroxide or its solutions, calcium oxide (lime) or its solutions, sodium carbonate or its solutions, potassium carbonate or its solutions, or mixtures of any of these may be selected, for example. In some embodiments, the strong base may comprise sodium hydroxide. For example, a 50% sodium hydroxide solution may be used to adjust the pH. In other exemplary embodiments, a less concentrated solution (*e.g*., 25% sodium hydroxide) may be used.

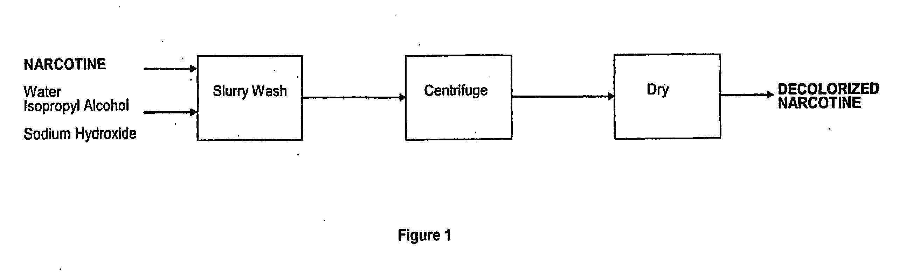

An exemplary process for the removal of impurities from a Narcotine product is shown in Figure 1. For example, the Narcotine may be slurried in an aqueous isopropanol solution. In some embodiments, the concentration of Narcotine may be from 30 to 40 grams ' per 100 ml isopropanol solution. The crude Narcotine may be broken into a powder using, *e.g*., a Stokes granulator or equivalent. After the pH of the suspension is adjusted using a strong base, *e*.*g*., sodium hydroxide, the suspension may be stirred. Although the suspension may be stirred for any amount of time, it is preferable to stir the suspension for a minimum of 15 minutes. The purified Narcotine may then be collected, *e.g*., in a filter or by using a centrifuge.

The process of the present invention may have a yield of at least 90% Narcotine, more preferably at least 95% Narcotine; most preferably at least 98% Narcotine.

According to exemplary embodiments, at least about 80% of the color may be removed from the Narcotine. In other exemplary embodiments, the process of the present invention may be used to remove at least about 90% of the color from the Narcotine. Ideally, the color is reduced to less than 0.6 abs., preferably less than 0.4 abs., most preferably less than 0.2 abs., using spectrophotometric analysis at 450 nm, with a sample being prepared as follows:
Cell Length = 1cm
wt of sample = 0.8 grams
volume of sample = 20 ml
solvent = 10% phosphoric acid
Temperature = Ambient
Wavelength = 450 nm
Reference = 10% phosphoric acid was used as a blank
Samples were filtered through a 0.45um Acrodisc prior to filling the cell for color determination.

In other exemplary embodiments, using the process of the present invention at least about 85% of Papaverine and Thebaine may be removed from the Narcotine, more preferably at least about 95% of Papaverine and Thebaine may be removed from the Narcotine. In still other exemplary embodiments, using the process of the present invention at least about 85% of the "Noscapine Analog" may be removed from the Narcotine.

In some embodiments, the Narcotine product will be from 68% - 99% pure after using the process of the present invention. In further embodiments, the Narcotine product will be from 90% - 99% pure after using the process of the present invention.

Thus, the present invention provides a novel process that may be used to purify Narcotine to form Noscapine. In some embodiments, after using the process of the present invention, the Narcotine may be further treated with methods known in the art. For example, the Narcotine may be treated with activated carbon and precipitated with ammonium hydroxide in isopropanol and water. Thus, the process of the present invention may be used alone or in combination with other purification techniques to remove impurities from Narcotine.

The following examples are merely illustrative of the present invention and should not be construed as limiting the scope of the invention in any way as many variations and equivalents that are encompassed by the present invention will become apparent to those skilled, in the art upon reading the present disclosure.

### EXAMPLES

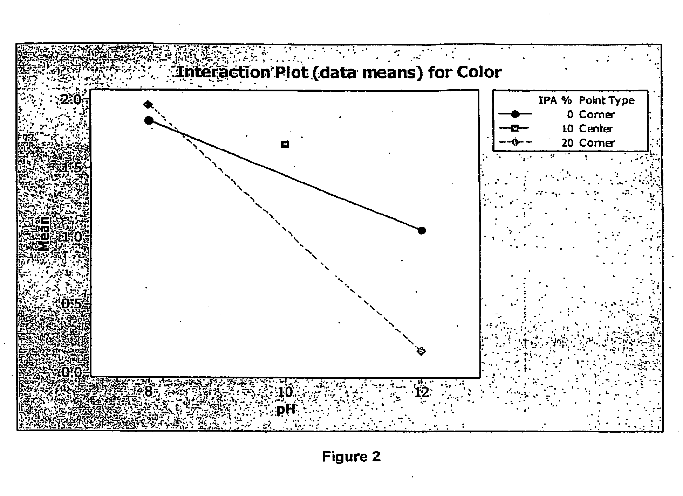

PROCEDURE: Experiment #1. Narcotine samples were purified with aqueous isopropanol solution of 0, 10%, and 20% v/v; and pH 8, 10, and 12. The steps that were used include: (1) Granulating the Narcotine into a coarse powder, (2) Adding the Narcotine (10 grams) to a flask provided with a magnetic stirrer, then adding isopropanol - water (200 ml), and adjusting the pH by adding sodium hydroxide solution, followed by stirring for 15 minutes at room temperature (18 - 25 °C); (3) The suspension was then filtered to collect the solids and washed with 20 ml of the same concentration of isopropanol as in the slurry wash; (4) The product was dried under vacuum at 60 °C. The results are summarized in Tables 1 and 2. An interaction plot is presented in Fig. 2.

**Table 1**

| Run | pH | Isopropanol Conc. (v/v) | Yield (g) | Color | Remarks |
|---|---|---|---|---|---|
| 1-1 | 8 | 0% | 9.00 | Brown | Slow Filtration |
| 1-2 | 8 | 20 | 8.76 | Brown | Fast Filtration |
| 1-3 | 12 | 20 | 9.03 | v. lt. tan/cream | |
| 1-4 | 10 | 10 | 9.11 | Brown | |
| 1-5 | 12 | 0 | 9.08 | Tan | Fast filtration |
| 1-6 | 10 | 10 | 9.51 | Brown | |

**Table 2**

| Run | pH | Isopropanol Conc. (v/v) | Yield (g) | Yield (% ANA) | Color | Assay % | | |
|---|---|---|---|---|---|---|---|---|
| | | | | | | ANA | APA | ATA |
| 1-1 | 8 | 0% | 9.00 | 90.46 | 1.848 | 88.38 | 2.12 | 0.18 |
| 1-2 | 8 | 20 | 8.76 | 90.50 | 1.96 | 90.84 | 0.5 | 0.11 |
| 1-3 | 12 | 20 | 9.03 | 94.57 | 0.155 | 92.09 | 0.25 | 0.01 |
| 1-4 | 10 | 10 | 9.11 | 90.16 | 1.594 | 87.02 | 1.19 | 0.09 |
| 1-5 | 12 | 0 | 9.08 | 92.12 | 1.053 | 89.21 | 1.7 | 0.12 |
| 1-6 | 10 | 10 | 9.51 | 97.00 | 1.764 | 89.69 | 1.0 | 0.11 |
| Crude | | | 10.00 | | 2.592 | 87.93 | 2.35 | 0.38 |

| | | | | | | | | |
|---|---|---|---|---|---|---|---|---|
| ANA = Anhydrous Narcotine Assay, APA = Anhydrous Papaverine Assay, ATA = Anhydrous Thebaine Assay | | | | | | | | |

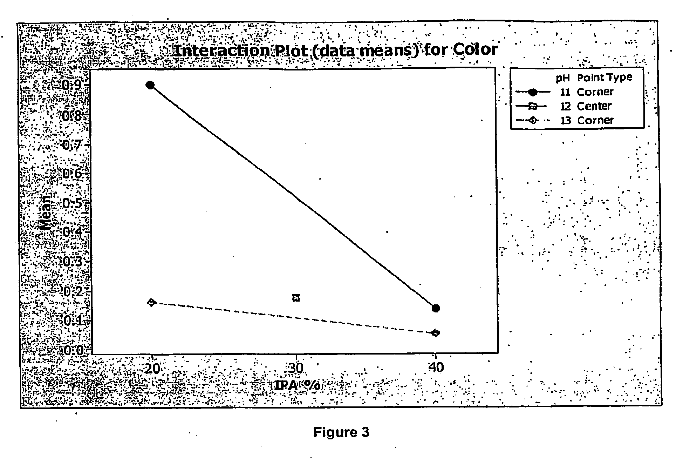

PROCEDURE: Experiment #2. Narcotine samples were purified with alcohol concentrations of 20%, 30% and 40% v/v; and pH of 11, 12, and 13. (1) The Narcotine was pulverized with a mortar and pestle, and then stirred to obtain a uniform feed for each experiment. (2) Narcotine (30.00 grams) and isopropanol - water (100 ml) was added to a flask provided with a magnetic stir bar. The pH was adjusted by adding sodium hydroxide solution. The slurry was stirred for fifteen minutes at room (18 - 25 °C) temperature. (3) The suspension was filtered, and the isolated solids were washed with 50 ml of the isopropanol solution of the same concentration as the wash. (4) The solids were dried under vacuum. The results are presented in Table 3. An interaction plot is presented in Figure 3.

**Table 3**

| Run | pH | Isopropanol Conc. (v/v) | Yield (g) | Yield (% ANA) | Color | Assay % | | |
|---|---|---|---|---|---|---|---|---|
| | | | | | | ANA | APA | ATA |
| 2-1 | 11 | 40 | 27.08 | 95.75 | 0.14 | 96.09 | 0.14 | 0.01 |
| 2-2 | 12 | 30 | 27.73 | 99.56 | 0.18 | 97.58 | 0.15 | 0.01 |
| 2-3 | 13 | 40 | 27.19 | 99.34 | 0.05 | 99.29 | 0.12 | 0.01 |
| 2-4** | 12 | 30 | 28.67* | 103.97 | 0.17 | 98.56 | 0.15 | 0.01 |
| 2-5 | 13 | 20 | 27.79 | 99.40 | 0.16 | 97.21 | 0.24 | 0.02 |
| 2-6 | 11 | 20 | 28.61 * | 104.97 | 0.90 | 99.71 | 0.44 | 0.06 |
| 2-4-rerun | 12 | 30 | 28.37* | 101.84 | 0.2 | 97.56 | 0.15 | 0.01 |
| Crude | JB183 | | 30.00 | | 2.043 | 90.59 | 2.18 | 0.38 |

PROCEDURE: Experiment #3. In further experiments, purification of Narcotine with ammonium hydroxide was compared to purification with sodium hydroxide. (1) Narcotine was pulverized with a mortar and pestle. (2) 22.50 grams of Narcotine and 75 ml isopropanol - water were added to a flask with a magnetic stirrer. The pH was adjusted by adding the base (ammonium hydroxide or sodium hydroxide solution) and stirred for 15 minutes at room temperature. (3) The suspension was filtered, and the solids were washed with 38 ml isopropanol - water with the same concentration as the slurry wash. (4) The solids were dried under vacuum. The results are shown in Table 4.

**Table 4**

| Run | pH | Isopropanol Conc. (v/v) | Yield (g) | Yield (% ANA) | Color | Assay % | | |
|---|---|---|---|---|---|---|---|---|
| | | | | | | ANA | APA | ATA |
| 3-1 NH₄OH | 11.79 | 30 | 19.50 | 94.7 | 0.732 | 89.2 | 0.00 | 1.81 |
| 3-2 NaOH | 11.8 | 30 | 19.14 | 94.0 | 0.184 | 90.2 | 0.00 | 0.74 |
| Crude | | | 22.50 | | 1.969 | 81.6 | 0.67 | 4.33 |

PROCEDURE: Experiment #4. Samples of Narcotine were also purified using the process of the present invention. Two batches of Narcotine were used: Batch A for Run 4-1 and Batch B for Run 4-2 and 4-3 (both were pulverized with a mortar and pestle, and blended in a bottle before use.) Tests were performed with 30 grams per 100 ml aqueous isopropanol solution (Run 4-1 and 4-2) and 40 grams per 100 ml aqueous isopropanol solution (Run 4-3.) Runs 4-1 and 4-2 were performed with 22.5 grams of Narcotine and 75 ml of aqueous isopropanol solution. Run 4-3 was performed with 30.00 grams of Narcotine and 75 ml aqueous isopropanol solution. In all cases, Narcotine and isopropanol - water solutions were added to a flash provided with a magnetic stirrer, the pH was adjusted with sodium hydroxide solution, and the suspensions were stirred for 15 minutes at room temperature. The suspensions were subsequently filtered and washed with isopropanol - water of the same concentration as the slurry wash. The solids were dried under vacuum. Despite the very high impurity concentration in the Narcotine, there was a 99% removal of Thebaine and Papaverine, and the yields of ANA were 95% - 105%. See Table 5.

**Table 5**

| Run | pH | Isopropanol 1 Conc. (v/v) | Yield (g) | Yield (% ANA) | Color | Assay % | | |
|---|---|---|---|---|---|---|---|---|
| | | | | | | ANA | APA | ATA |
| Crude | Batch A | | 22.50 | | 1.969 | 81.6 | 0.67 | 4.33 |
| 4-1 | 13 | 40 % | 18.70 | 94.7 | 0.051 | 93.0 | 0.04 | 0.04 |
| | | | | | | | | |
| Crude | Batch B | | 22.5/30.0 | | 5.290 | 69.7 | 1.85 | 6.22 |
| 4-2 | 13 | 40 | 16.97 | 104.9 | 0.109 | 96.9 | 0.02 | 0.07 |
| 4-3 | 13 | 40 | 22.91 | 102.8 | 0.121 | 93.9 | 0.02 | 0.38 |

PROCEDURE: Experiment #5. The effectiveness of a second slurry wash on intermediate - purity Narcotine was also tested. Again, the Narcotine was pulverized with a mortar and pestle, and blended in a bottle before use. Narcotine and isopropanol - water were added to a flask provided with a magnetic stirrer, and the pH was adjusted with sodium hydroxide solution, and stirred for 15 minutes. The suspension was filtered, and the solids washed with isopropanol - water. The solids were dried under vacuum. Run 5-1 was performed with 30.00 grams of Narcotine and 100 ml of 40% aqueous isopropanol solution at pH 13. The wash volume was 50 ml. Run 5-2 and 5-3 were performed with 30.00 grams of Narcotine, and 75 ml of 40% aqueous isopropanol solution, again at pH 13. The wash volumes were 38 ml. Run 5-3 was also slurry washed under the same conditions (75 ml 40% aqueous isopropanol solution, pH 13, and 38 ml wash) a second time. See Table 6.

**Table 6**

| Run | pH | Isopropanol Conc. (v/v) | Yield (g) | Yield (% ANA) | Color | Assay % | | |
|---|---|---|---|---|---|---|---|---|
| | | | | | | ANA | APA | ATA |
| 5-1 | 13 | 40% | 28.20 | 100.4 | 0.044 | 100.58 | 0.031 | 0.072 |
| 5-2 | 13 | 40 | 28.46 | 99.56 | 0.049 | 98.82 | 0.034 | 0.084 |
| 5-3 | 13 | 40 | 28.40 | 102.2 | 0.036 | 101.7 | 0.024 | 0.061 |
| Crude | Batch C | | 30.00 | 94.16 | 2 | 94.16 | 0.121 | 0.285 |

PROCEDURE: Experiment #6. The use of an aqueous isopropanol solution with varied alcohol concentration was also tested. In particular, alcohol concentration of 65% v/v isopropanol (Run 7-1) and 50% v/v isopropanol (Run 7-2) were tested. Narcotine was pulverized with a mortar and pestle. Narcotine (35 grams) and isopropanol - water (100 ml) were charged to a flask provided with a magnetic stirrer. The pH was adjusted with sodium hydroxide solution, and stirred for 15 minutes. The suspension was filtered, and the solids washed with the same concentration isopropanol - water as used in the slurry wash. The solids were dried under vacuum. The results are shown in Table 7.

**Table 7**

| Run | pH | Isopropanol Conc. (v/v) | Yield (g) | Yield (% ANA) | Color | Assay % | | |
|---|---|---|---|---|---|---|---|---|
| | | | | | | ANA | APA | ATA |
| 7-1 | 13 | 65% | 33.04 | 97.9 | 0.371 | 97.67 | 0.044 | 0.067 |
| 7-2 | 13 | 50% | 33.17 | 98.7 | 0.031 | 98.11 | 0.032 | 0.054 |
| Crude | Batch C | | | | 2 | | | |

PROCEDURE: Experiment #7. A set of comparative examples was run according to the following conditions. Crude Narcotine was decolorized by slurry washing 30.0 grams each in 100 ml isopropyl alcohol - water. The first experiment, 8-1 was done with 30% isopropyl alcohol (v/v), and the pH was adjusted to 9.0 with sodium hydroxide. The second experiment, 8-2 was done with 40% isopropyl alcohol (v/v), and the pH was adjusted to 8.0 with sodium hydroxide. In each case the sample was stirred for 15 minutes, filtered, and washed with 25 ml isopropyl alcohol - water of the same concentration as the slurry wash. The solids were dried under vacuum. The results are shown in Table 8.

**Table 8 (Comparative)**

| Run | pH | Isopropanol Conc. (v/v) | Color | Assay % | | |
|---|---|---|---|---|---|---|
| | | | | ANA | APA | ATA |
| 8-1 | 9 | 30% | 1.437 | 100.76 | 0.13 | 0.08 |
| 8-2 | 8 | 40% | 0.924 | 100.86 | 0.06 | 0.05 |
| Crude | JB244 | | 2.142 | | | |

While the invention has been depicted and described by reference to exemplary embodiments of the invention, such a reference does not imply a limitation on the invention, and no such limitation is to be inferred. The invention is capable of considerable modification, alteration, and equivalents in form and function, as will occur to those ordinarily skilled in the pertinent arts having the benefit of this disclosure. The depicted and described embodiments of the invention are exemplary only, and are not exhaustive of the scope of the invention.

## Claims

1. A process for the removal of impurities from a Narcotine product comprising:
(a) forming a suspension of an aqueous isopropanol solution and the Narcotine product, wherein the isopropanol concentration of the solution is 20% to 70% by volume; and
(b) adjusting the pH of the suspension with a strong base to a pH of 10 to 14, wherein impurities are removed from the Narcotine product.

2. The process of claim 1 further comprising stirring the suspension for at least 15 minutes after adjusting the pH.

3. The process of claim 2 wherein the suspension is stirred at a temperature from 18°C to 25°C.

4. The process of claim 1 or 3 further comprising filtering the suspension to separate the impurities from the Narcotine product.

5. The process of claim 1 further comprising centrifuging the suspension to separate the impurities from the Narcotine product.

6. The process of claim 4 further comprising washing the filtered suspension with the aqueous isopropanol solution.

7. The process of claim 1 wherein the isopropanol concentration of the solution is 30% to 60% by volume.

8. The process of claim 1 wherein the isopropanol concentration of the solution is 30% to 50% by volume.

9. The process of claim 1 wherein the strong base is selected from the group consisting of sodium hydroxide, potassium hydroxide, calcium hydroxide, calcium oxide, sodium carbonate, potassium carbonate, or solutions or mixtures thereof.

10. The process of claim 1 wherein the strong base comprises sodium hydroxide.

11. The process of claim 10 wherein the strong base comprises a 50% sodium hydroxide solution.

12. The process of claim 10 wherein the strong base comprises a 25% sodium hydroxide solution.

13. The process of claim 1 wherein the aqueous solution is adjusted to a pH of 12 to 13.

14. The process of claim 1 wherein the Narcotine product is present in the suspension in an amount of from about 30 to about 40 grams per 100 ml aqueous isopropanol solution.

15. The process of claim 4 or 5 further comprising treating the Narcotine product with activated carbon.

16. The process of claim 15 further comprising precipitating the Narcotine product with ammonium hydroxide in isopropanol and water.

## Patentansprüche

1. Verfahren zur Entfernung von Verunreinigungen aus einem Narcotin-Produkt, umfassend:
(a) Bilden einer Suspension einer wässrigen Isopropanollösung und des Narcotin-Produkts, wobei die Isopropanolkonzentration der Lösung 20 Volumenprozent bis 70 Volumenprozent beträgt, und
(b) Einstellen des pH der Suspension mit einer starken Base auf einen pH von 10 bis 14, wobei Verunreinigungen aus dem Narcotin-Produkt entfernt werden.

2. Verfahren gemäß Anspruch 1, das außerdem Rühren der Suspension für wenigstens 15 Minuten nach Einstellung des pH umfasst.

3. Verfahren gemäß Anspruch 2, wobei die Suspension bei einer Temperatur von 18°C bis 25°C gerührt wird.

4. Verfahren gemäß Anspruch 1 oder 3, das außerdem Filtrieren der Suspension umfasst, um die Verunreinigungen aus dem Narcotin-Produkt abzutrennen.

5. Verfahren gemäß Anspruch 1, das außerdem Zentrifugieren der Suspension umfasst, um die Verunreinigungen aus dem Narcotin-Produkt abzutrennen.

6. Verfahren gemäß Anspruch 4, das außerdem Waschen der filtrierten Suspension mit der wässrigen Isopropanollösung umfasst.

7. Verfahren gemäß Anspruch 1, wobei die Isopropanolkonzentration der Lösung 30 Volumenprozent bis 60 Volumenprozent beträgt.

8. Verfahren gemäß Anspruch 1, wobei die Isopropanolkonzentration der Lösung 30 Volumenprozent bis 50 Volumenprozent beträgt.

9. Verfahren gemäß Anspruch 1, wobei die starke Base aus der Gruppe, bestehend aus Natriumhydroxid, Kaliumhydroxid, Calciumhydroxid, Calciumoxid, Natriumcarbonat, Kaliumcarbonat oder Lösungen oder Gemischen davon, ausgewählt wird.

10. Verfahren gemäß Anspruch 1, wobei die starke Base Natriumhydroxid umfasst.

11. Verfahren gemäß Anspruch 10, wobei die starke Base eine 50%-ige Natriumhydroxidlösung umfasst.

12. Verfahren gemäß Anspruch 12, wobei die starke Base eine 25%-ige Natriumhydroxidlösung umfasst.

13. Verfahren gemäß Anspruch 1, wobei die wässrige Lösung auf einen pH von 12 bis 13 eingestellt wird.

14. Verfahren gemäß Anspruch 1, wobei das Narcotin-Produkt in der Suspension in einer Menge von etwa 30 bis etwa 40 Gramm pro 100 ml wässrige Isopropanollösung vorliegt.

15. Verfahren gemäß Anspruch 4 oder 5, das außerdem Behandeln des Narcotin-Produkts mit Aktivkohle umfasst.

16. Verfahren gemäß Anspruch 15, das außerdem Präzipitieren des Narcotin-Produkts mit Ammoniumhydroxid in Isopropanol und Wasser umfasst.

## Revendications

1. Procédé pour l'élimination des impuretés d'un produit de narcotine comprenant les étapes consistant à (a) préparer une suspension d'une solution aqueuse d'isopropanol avec le produit de narcotine, la concentration en isopropanol de la solution étant d'environ 20% à environ 70% en volume, et (b) régler le pH de la suspension avec une base forte jusqu'à obtenir un pH d'environ 10 à 14, les impuretés étant éliminées du produit de narcotine.

2. Procédé selon la revendication 1 comprenant en outre une étape consistant à agiter la suspension pendant au moins 15 minutes après avoir réglé le pH.

3. Procédé selon la revendication 2, dans lequel la suspension est agitée à une température variant de 18°C à 25°C.

4. Procédé selon la revendication 1 ou 3, comprenant en outre une étape consistant à filtrer la suspension pour séparer les impuretés du produit de narcotine.

5. Procédé selon la revendication 1 comprenant en outre une étape consistant à centrifuger la suspension pour séparer les impuretés du produit de narcotine.

6. Procédé selon la revendication 4 comprenant en outre une étape consistant à laver la suspension filtrée avec la solution aqueuse d'isopropanol.

7. Procédé selon la revendication 1, dans lequel la concentration en isopropanol de la solution varie de 30% à 60% en volume.

8. Procédé selon la revendication 1, dans lequel la concentration en isopropanol de la solution varie de 30% à 50% en volume.

9. Procédé selon la revendication 1, dans lequel la base forte est choisie parmi le groupe consistant en hydroxyde de sodium, hydroxyde de potassium, hydroxyde de calcium, oxyde de calcium, carbonate de sodium, carbonate de potassium, ou solutions ou mélanges de ceux-ci.

10. Procédé selon la revendication 1, dans lequel la base forte comprend l'hydroxyde de sodium.

11. Procédé selon la revendication 10, dans lequel la base forte comprend l'hydroxyde de sodium en solution à 50%.

12. Procédé selon la revendication 10, dans lequel la base forte comprend l'hydroxyde de sodium en solution à 25%.

13. Procédé selon la revendication 1, dans lequel la solution aqueuse est réglée à un pH de 12 à 13.

14. Procédé selon la revendication 1, dans lequel le produit de narcotine est présent dans la solution en une quantité variant d'environ 30 à environ 40 grammes par 100 ml de la solution aqueuse d'isopropanol.

15. Procédé selon la revendication 4 ou 5 comprenant en outre une étape consistant à traiter le produit de narcotine du charbon actif.

16. Procédé selon la revendication 15 comprenant en outre l'étape consistant à précipiter le produit de narcotine avec de l'hydroxyde d'ammonium dans de l'isopropanol et de l'eau.
